(19) Europäisches Patentamt / European Patent Office / Office européen des brevets

(11) **EP 1 025 130 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**24.08.2016 Patentblatt 2016/34**

(45) Hinweis auf die Patenterteilung:
**04.08.2004 Patentblatt 2004/32**

(21) Anmeldenummer: **98965627.7**

(22) Anmeldetag: **12.10.1998**

(51) Int Cl.:
***C08B 11/12*** (2006.01)    ***A61L 15/28*** (2006.01)

(86) Internationale Anmeldenummer:
**PCT/EP1998/006451**

(87) Internationale Veröffentlichungsnummer:
**WO 1999/020657 (29.04.1999 Gazette 1999/17)**

(54) **WEITGEHEND FASERFREIE CELLULOSEETHER MIT VERBESSERTER WASSERRETENTION, VERFAHREN ZUR HERSTELLUNG UND IHRE VERWENDUNG**

ESSENTIALLY FIBRE-FREE CELLULOSE ETHER WITH IMPROVED WATER RETENTION, METHOD FOR THE PRODUCTION AND USE THEREOF

ETHER DE CELLULOSE EXEMPT DE FIBRES, A RETENTION D'EAU AMELIOREE, SON PROCEDE DE FABRICATION ET SON UTILISATION

(84) Benannte Vertragsstaaten:
**AT BE CH DE DK ES FI FR GB GR IE IT LI NL PT SE**

(30) Priorität: **20.10.1997 DE 19746264**

(43) Veröffentlichungstag der Anmeldung:
**09.08.2000 Patentblatt 2000/32**

(73) Patentinhaber: **Dow Global Technologies LLC Midland, MI 48674 (US)**

(72) Erfinder:
• **LANGE, Werner**
  **D-27374 Visselhövede (DE)**
• **SCHRIEWER, Bernd**
  **D-29664 Walsrode (DE)**
• **LAMPERT, Friedrich-Karl**
  **D-29699 Bomlitz (DE)**
• **OPPERMANN, Wilhelm**
  **D-29664 Walsrode (DE)**
• **PANNEK, Jörn-Bernd**
  **D-29683 Fallingbostel (DE)**
• **KIESEWETTER, René**
  **D-29614 Soltau (DE)**

(74) Vertreter: **f & e patent**
**Fleischer, Engels & Partner mbB, Patentanwälte**
**Braunsberger Feld 29**
**51429 Bergisch Gladbach (DE)**

(56) Entgegenhaltungen:
EP-B- 0 201 895    DD-A1- 233 377
DE-A- 1 418 238    DE-A- 1 801 553
DE-A- 2 151 973    DE-A1- 2 400 879

US-A- 3 678 031    US-A- 4 579 943
US-A- 5 618 800

• SCHOICHIRO YANO: "Dynamic viscoelastic properties of carboxymethylcellulose during isothermal water sorption," POLYMER., Bd. 34, Nr. 10, 1. Mai 1993, Seiten 2528-2532, XP002100708 GB
• PATENT ABSTRACTS OF JAPAN vol. 16, no. 106 (C-919), 16. März 1992 & JP 03 279471 A (TOYOBO CO LTD), 10. Dezember 1991 & DATABASE WPI Week 9204 Derwent Publications Ltd., London, GB; AN 30723
• L. BRANDT: 'Ullmann's Encyclopedia of Industrial Chemistry', teil A5 1987 Seiten 461 - 488
• R. LAPASIN ET AL.: 'Rheology of Industrial Polysaccharides: Theory and Applications', 1995, BLACKIE ACADEMIC & PROFESSIONAL, LONDON Seiten 398 - 399
• D. J. SIKKEMA ET AL.: '"(Carboxymethyl) cellulose with Xanthan Gum Like Rheology"' MACROMOLECULES Bd. 22, Nr. 1, 1989, Seiten 364 - 366
• J. G. WESTRA: 'Rheology of (Carboxymethyl)cellulose with Xanthan Gum Properties' MACROMOLECULES Bd. 22, 1989, Seiten 367 - 370
• J.V. HERRÁEZ-DOMINGUEZ ET AL.: 'Rheological characterization of two viscosity grades of methylcellulose: an approach to the modeling of the thixotropic behaviour' COLLOID POLYM SCI Bd. 284, 2005, Seiten 86 - 91

- T. MEZGER: 'Das Rheologie-Handbuch', Bd. 2, 2000, CURT R. VINCENTZ VERLAG, HANNOVER Seite 57
- ISO 760
- T. MEZGER: 'Das Rheologie-Handbuch', 2000, CURT R. VINCENTZ VERLAG, HANNOVER Seiten 152 - 153
- Walocel® M - Methylcellulosen für Putz- und Mauermörtel, Broschür der Wolff Walsrode AG, Februar 1996, und
- Culminal® - Methylcellulose und Methylhydroxypropylcellulose, Broschüre der Henkel KGaA, Juni 1980

**Beschreibung**

Die Erfindung bezeichnet ein Verfahren zur Herstellung von weitgehend faserfreien Celluloseethern mit überwiegend elastischen Eigenschaften, entsprechende Celluloseether sowie ihre Verwendung als superabsorbierendes Material und als Hilfsmittel zur Einstellung einer geeigneten Rheologie und Wasserrückhaltung für die Bereiche Kosmetik, Pharmazie und Lebensmittel sowie ihren Einsatz für technische Anwendungen, wie z. B. als Additiv zum Abdichten von Kabeln (Telekommunikationskabel u. ä.).

Unter Superabsorbern sollen im Sinne dieser Erfindung Produkte verstanden werden, die als Pulver oder Granulate in der Lage sind, Flüssigkeit (Wasser, Urin, Wundsekret, Blut u. ä.) aufzunehmen und auch unter Belastung bei einem Druck von 0,1 psi - 1 psi, wie er z. B. beim Tragen von Pflastern, Windeln, Binden und Hygieneartikeln aller Art sowie Zahnhaftcremes auftritt, zurückzuhalten.

Nach dem Stand der Technik werden pulverförmige synthetische Superabsorber auf Polyacrylatbasis für Hygieneprodukte (z. B. Binden etc.) eingesetzt. Ihre superabsorbierenden Eigenschaften liegen bei ca. 50 g Flüssigkeit pro g Polymer. Die Produkte gelten jedoch als biologisch nicht abbaubar. Betrachtet man lediglich den Anteil sog. Wegwerfwindeln am gesamten Hausmüllaufkommen in Deutschland, der derzeit bei ca. 2 - 3 % liegt, so ist es verständlich, dass man nach Möglichkeiten sucht, synthetische Produkte durch biologisch abbaubare oder kompostierbare Stoffe zu ersetzen und diese mit zumindest gleichbleibenden technischen Qualitäten, z. B. superabsorbierenden Eigenschaften, auszustatten.

Herkömmliche unvernetzte Carboxymethylcellulosen (nachfolgend auch als CMC bezeichnet), die in Gegenwart einer Lauge, wie z. B. Natronlauge, aufgeschlossen und mit einem Veretherungsmittel, wie z. B. Monochloressigsäure, in einem Gemisch eines organischen Suspensionsmittels und Wasser verethert werden, werden herkömmlicherweise als nicht superabsorbierende Produkte angesehen (siehe hierzu R. L. Whistler in "Industrial Gums", Seite 704 ff (2. Auflage 1973)). Es handelt sich dabei üblicherweise um unvernetzte Carboxymethylcellulosen, die mit einem Gemisch aus einem für CMC unlöslichen Suspensionsmittel und Wasser gewaschen werden und deren Faserstruktur noch unter dem Mikroskop deutlich zu erkennen ist (siehe hierzu US-2715124). Eine so hergestellte Carboxymethylcellulose hat je nach Einsatz und Polymerisationsgrad (DP) des verwendeten Celluloserohstoffes (Holzzellstoff, Baumwollinters, Rohlinters etc.) unterschiedlich verdickende, aber in der Regel keine absorbierenden oder gar superabsorbierenden Eigenschaften. Es hat daher nicht an Versuchen gefehlt, die normalerweise wasserlösliche Carboxymethylcellulose In eine unlösliche Form zu überführen und die Absorptionseigenschaften durch Einsatz von Vernetzungsmit-teln zu verbessern. Als Vernetzungsmittel werden z. B. 1,2-Dichlorethan, Epichlorhydrin, Aldehyde, wie z. B. Formaldehyd, oder komplexbildende Metallsalze, wie z. B. Chromverbindungen, beschrieben (JP 04161431-A, J 63037143 A, US 4952550, RD 349022 A). Es hat auch nicht an Versuchen gefehlt, Abmischungen von modifizierten Kohlenhydratpolymeren mit in Wasser quellbaren synthetischen Polymeren, wie z. B. vernetzten Polyacrylamiden bereitzustellen (EP 0131090, US 4,021,257. US 4,110,226, US 3,574,188, EP 0056360. DE 3929400, DE 4328190 A1 und DE 4206857 A1). Von besonderem Nachteil sind hier jedoch ökotoxikologische Aspekte bei der Herstellung, Anwendung und Deponierung der vernetzten Polymere. So sind beim Einsatz von mit chlororganischen Verbindungen oder Aldehyden vernetzten Polymeren besondere verfahrenstechnische Maßnahmen zum Schutz von Mensch und Umwelt erforderlich. Der Einsatz entsprechend vernetzter Carboxymethylcellulosen in z. B. Hygieneprodukten (z. B. Windeln, Binden), bei denen die CMC mittelbar oder unmittelbar mit der Haut in Berührung kommt, kann darüber hinaus z. B. allergische Reaktionen oder Schädigungen des vegetativen Nervensystems hervorrufen. Bei der Deponierung kann es schließlich durch Auswaschprozesse zur Kontaminierung des Grundwassers kommen.

In der Anmeldung EP 0 201 895 B1 wird ein Verfahren zur Herstellung einer weitgehend nichtfaserigen, superabsorbierenden CMC beschrieben, bei der das superabsorbierende Produkt dadurch gewonnen wird, dass die CMC in Wasser gelöst und durch Zusetzen eines Nichtlösungsmittels (z. B. Aceton oder Isopropanol) erhalten wird.

Das Auflösen einer bereits gefertigten CMC oder eines CMC-Kuchens in Wasser und das nachträgliche Ausfällen durch Zusetzen eines Nichtlösungsmittels für CMC stellt jedoch einen zusätzlichen verfahrenstechnischen Schritt dar, der zu einer Verteuerung des Verfahrens führt.

Aufgabe der vorliegenden Erfindung war es daher, Celluloseether, insbesondere eine Carboxymethylcellulose, mit verbesserten absorbierenden, insbesondere superabsorbierenden Eigenschaften bereitzustellen, ohne dabei toxische oder ökologisch bedenkliche Substanzen zu verwenden. Verfahrenstechnisch sollte das Produkt einfach und kostengünstig herstellbar sein.

Gegenstand der Erfindung sind daher weitgehend faserfreie Celluloseether, dadurch gekennzeichnet, dass der Gesamtwasseranteil bei der Herstellung der Celluloseether bei 11-28 Vol.-%, bezogen auf Cellulose, Suspensionsmittel, Natriumhydroxid und Veretherungsmittel, liegt und zur Herstellung der Celluloseether Mengen von Alkali von mindestens 1,8 Mol-2,6 Mol/Mol Glucoseeinheit verwendet werden und wässrige Lösungen in Konzentrationen von maximal 0,5 Gew.-% Werte für den Verlustfaktor (tan $\delta$) von < 1,0 bei einer Kreisfrequenz von 1 Hz zeigen.

Überraschenderweise konnte gezeigt werden, dass lediglich durch Änderung der Alkalisierungsbedingungen bei der Herstellung entsprechender Celluloseether der Stand der Technik in technischer und ökotoxikologischer Hinsicht verbessert werden kann. Es zeigte sich, dass in wässriger Lösung die in der vorliegenden Anmeldung beschriebenen Produkte auch ohne Vernetzungsreagenzien hochfeste Gele mit verbesserter Wasserrückhaltung ausbilden. Bei Verwendung als Pulver wird z. B. in Wundpflastern, Windeln, Binden, Zahnprothesenhafteremes etc. eine erheblich verbesserte Wasserrückhaltung gegenüber Flüssigkeiten, wie z. B. Blut, Speichel, Wundsekret, Urin u.ä. beobachtet. Dadurch, dass toxische Substanzen weder im Produkt selbst noch bei der verfahrenstechnischen Herstellung zum Einsatz kommen, ist neben technischen Anwendungen auch der unbedenkliche Einsatz dieser Stoffe für Lebensmittel-, Kosmetik- und Pharmaanwendungen möglich. Darüber hinaus zeigen die erfindungsgemäßen wasserlöslichen Celluloseether gegenüber dem Stand der Technik andere rheologische, insbesondere elastische Eigenschaften, durch die eine Differenzierung gegenüber herkömmlichen Celluloseethern, insbesondere CMCs, möglich ist. Dies hat zur Folge, dass die erfindungsgemäß beanspruchten Produkte in Anwendungsbereichen - allein oder in Kombination mit zusätzlichen Hilfsmitteln auch dort einsetzbar sind, in denen herkömmliche Celluloseether Defizite - z.B. durch fehlende Fließgrenzen - zeigen (z.B. im Schlitzwand- und Bohrpfahlwandbau u.ä. oder bei Anstrichmitteln (Dispersions- oder Silikatfarben u.ä.)).

Aufgrund der sehr hohen Viskositätsergiebigkeiten ist es zudem möglich, die Einsatzmengen an Celluloseethern in bestehenden Anwendungen reduziert werden können, ohne dabei technische Nachteile in Kauf nehmen zu müssen. Dadurch wird der Forderung entsprochen, den Anteil an Zusatzmitteln in verschiedenen Formulierungen für z. B. Kosmetika (z. B. Haarschampoos etc.) aus toxikologischen Gründen weiter zu reduzieren. Für technische Anwendungen, z. B. dem Schlitzwand- und Tunnelbau, geht der reduzierte Einsatz der erfindungsgemäß beanspruchten Produkte mit verbesserten ökotoxikologischen Werten, z. B. verringerten CSB- und TOC-Werten im Boden, Abwasser etc, einher.

Durch die vorliegende Erfindung wird zugleich ein Verfahren zur Herstellung eines weitgehend faserfreien Celluloseethers, insbesondere einer Carboxymethylcellulose (CMC), mit überwiegend elastischen sowie superabsorbierenden Eigenschaften beschrieben. Der beanspruchte Celluloseether (insbesondere CMC) besitzt eine Saugfähigkeit von mindestens 30 g Flüssigkeit pro Gramm Celluloseether und eignet sich zur Einstellung einer geeigneten Rheologie, Wasserrückhaltung für die Bereiche Kosmetik, Pharmazie und Lebensmittel sowie für technische Anwendungen, wie z. B. für Anstrichmittel (z.B. Dispersions- oder Silikatfarben u.ä.) oder dem Tiefbau (Tunnelbau, Schlitzwandbau u. a.). Das Verfahren ist durch folgende Schritte gekennzeichnet:

1. Einsatz einer Cellulose mit einem Durchschnittspolymerisationsgrad (DP) von mindestens 1.000, insbesondere von > 2.000 - 3.500 unter Verwendung hierfür geeigneter Rohstoffe, wie z. B. Holz- und Nadelholzzellstoffen, Linters oder Rohlinters sowie Gemischen hieraus.

2. Verwendung eines wässrig-organischen Suspensionsmittels zur Herstellung eines Celluloseethers, bevorzugt von Carboxymethylcellulose, Sulfoethylcellulose, Methyl-oderMethyl-Hydroxyalkylcelluloseether (MHEC, MHPC) oder Hydroxyalkylcelluloseether (HEC, HPC)), wie vorzugsweise Isopropanol-Wasser, Aceton-Wasser, Methanol-Wasser, Ethanol-Wasser oder tertiär-Butanol-Wasser oder Gemischen hiervon mit einem Gesamtwasseranteil - bezogen auf Cellulose, Suspensionsmittel, Natriumhydroxid und Veretherungsmittel (wie z.B. Chloressigsäure oder Vinylsulfonsäure o.a.) - von mindestens 11 Vol.-%, höchstens 28 Vol-%, vorzugsweise 12,5 - 25 Vol.-%, insbesondere 13 - 20 Vol.-%, besonders bevorzugt von 13,5- 18 Vol.-% und einer Menge an Alkali, wie z. B. Natriumhydroxid, von mindestens 1,8 Mol - 2,6 Mol, vorzugsweise 2,0 - 2,5 Mol, insbesondere 2,1 - 2,4 Mol/Mol Glucoseeinheit.

3. Herstellung eines superabsorbierenden Celluloseethers, insbesondere von CMC, nach mindestens einem der vorgenannten Punkte, dadurch gekennzeichnet, dass für die Herstellung des Celluloseethers, insbesondere einer Carboxymethylcellulose, eine Menge an Veretherungsmittel, insbesondere Monochloressigsäure, von 0,4 - 2,5 Mol, insbesondere von 0,5 - 1,8 Mol, vorzugsweise von 0,6 - 1,5 Mol/Mol Glucose benötigt wird.

4. Veretherung, Reinigung, Trocknung und Konfektionierung in üblicher und bekannter Weise, wobei der so erhaltene erfindungsgemäße Celluloseether, insbesondere CMC, einen Faseranteil von < 1 %, einen Durchschnittssubstitutionsgrad (DS) durch Ethergruppen, insbesondere Carboxymethylgruppen, von DS = 0,2 - 1,5, insbesondere von 0,3 - 1,2, eine Absorptionskapazität von > 30 g Flüssigkeit/Gramm Celluloseester, insbesondere > 35 g Flüssigkeit/Gramm Celluloseester, einen Gesamtsalzanteil (Natriumchlorid, Natriumglycolat) von < 1 % sowie eine durch Mahlung und Siebung eingestellte Sieblinie von 100 % < 2 mm, 100 % < 0,5 mm und mindestens 80 % < 0,075 mm hat.

Jede Anwendung macht es erforderlich, die physiko-chemischen Eigenschaften (Viskosität und Molekularität, Molekulargewichtsverteilung, Sieblinie, Rheologie, Substitution. Partikelmorphologie, Faseranteil u.a.) des jeweiligen Celluloseethers den speziellen anwendungstechnischen Erfordernissen anzupassen. Für die Bereitstellung eines saugfähigen Celluloseesters, insbesondere CMC bzw. einer solchen mit optimalen superabsorbierenden Eigenschaften, wie sie hier für z.B. für Wundpflaster, Windeln, Binden etc. beschrieben wird, müssen der Durchschnittspolymerisationsgrad (DP), der Durchschnittssubstitutionsgrad (DS), der Faseranteil sowie die Partikelmorphologie genau aufeinander abgestimmt werden.

Die Verwendung von Cellulosen oder Cellulosegemischen mit Durchschnittspolymerisationsgraden von > 1.000, insbesondere von > 2.000 - 3.500 ist erforderlich, weil die Celluloseether, insbesondere CMC, sonst zu niedrige Absorptionskapazitäten oder überhaupt keine saugfähigen Eigenschaften mehr aufweist. Ebenso zeigen Produkte mit grober Partikelgröße eine zu geringe Oberfläche und damit verbundene ungenügende Absorption.

Die Einstellung eines geeigneten Durchschnittssubstitutionsgrades (DS) durch Ethergruppen, insbesondere Carboxymethylgruppen, ist ebenso entscheidend. Ist der DS zu niedrig (< 0,2), ist das Produkt wasserunlöslich und nur wenig quellbar bzw. stark faserhaltig und besitzt nur geringe absorbierende Eigenschaften. Substitutionsgrade (DS) von > 1,5 führen hingegen bezüglich der Löslichkeit und Saugfähigkeit zu keiner weiteren Verbesserung der Produkteigenschaften. Hingegen wird die Veretherung aufgrund des Mehreinsatzes an Veretherungsreagenz bzw. wegen der ggf. mehrfachen Wiederholung des Veretherungsschrittes zunehmend unwirtschaftlich und kann aufgrund des höheren Salzanfalls oder einer verbesserten Löslichkeit der Celluloseether, insbesondere CMC, zu Problemen bei der Aufarbeitung durch verlängerte Waschcyclen oder durch Anlösen oder starkes Quellen des Celluloseethers, insbesondere der CMC, beim Einsatz wässriger Waschmedien führen. Zudem zeigt das Produkt mit ansteigendem Substitutionsgrad eine immer schlechter werdende biologische Abbaubarkeit.

Die Einstellung eines für eine hohe Absorption erforderlichen niedrigen Faseranteils von < 1 % erfolgt über den Einsatz geeigneter Mengen an Lauge, wie z. B. Natronlauge, und die Überführung von kristallinen in amorphe Bereiche der Cellulose sowie durch die Menge an Veretherungsmittel, wie z. B. Monochloressigsäure oder dem Natriumsalz, Vinylsulfonsäure, Methylchlorid, Hydroxyalkylierungsreagenzien wie Ethylenoxid oder Propylenoxid oder Gemischen hiervon.

Dem wässrig-organischen Suspensionsmittel (Slurry) kommt demnach die die Aufgabe zu, das Gemisch aus Wasser und Alkali bzw. das Veretherungsmittel im Reaktionsmedium zu verteilen und von über- zu unteralkalisierten Bereichen der Cellulose zu übertragen, um so eine weitgehend homogene Verteilung der Lauge und der nachfolgenden Veretherungsmittel sicherzustellen. Als wässrig-organische Suspensionsmittel werden bevorzugt Isopropanol-Wasser-, Aceton-Wasser-, Methanol-Wasser-, Ethanol-Wasser- oder tertiär-Butanol-Wassergemische oder binäre oder ternäre Gemische der vorgenannten Suspensionsmittel mit Wasser verwendet. Eine Beschränkung auf bestimmte Suspensionsmittel erfolgt hierbei nicht, da auch andere hier nicht explizit genannte Gemische erfolgreich einsetzbar sind (s. hierzu z.B. EP 0080678, EP 0161607, EP 0126959).

Die dem Suspensionsmittel zugesetzte Menge an Wasser dient dazu, ein ausreichendes Quellvermögen der Cellulose zu Beginn der Alkalisierung zu ermöglichen, um damit eine optimale Zugänglichkeit der Alkalicellulose für die eingesetzten Reagenzien zu gewährleisten (siehe z. B. US-PS 4,547,570). Dem Fachmann ist bekannt, dass ein zu hoher Anteil von Wasser im Suspensionsmittel unwirtschaftlich ist, da dies zu einer Verschlechterung der Ausbeute an Veretherungsreagenz führt (US-PS 4,547,570), so dass das Verfahren dadurch unnötig verteuert wird. Darüber hinaus steigt mit zunehmendem Wasseranteil im Suspensionsmittel der Anteil an Gelkörpem an, was zu Problemen bei der Aufarbeitung führen kann (siehe SU-B 553253; CA87,1977, Ref. 25055 f und Houben-Weyl S. 2054), so dass die Umsetzung aus verfahrenstechnischen und wirtschaftlichen Gründen nicht sinnvoll ist oder mit entsprechenden Nachteilen verbunden ist. Verfahrenstechnische Verbesserungen gingen daher bisher dahin, den für die Reaktion erforderlichen Wassergehalt während der Alkalisierungs- und Veretherungsphase niedrig zu halten, um so hohe Produktausbeuten sicherzustellen.

Überraschenderweise wurde nun festgestellt, dass dem Wasserhaushalt während der Veretherung bzw. Alkalisierung des Celluloseethers, insbesondere bei der Herstellung von Carboxymethylcellulose, eine überragende Bedeutung bei der Kontrolle der Wasserrückhaltung im fertigen Produkt sowie der für die Anwendung erforderlichen Rheologie zukommt.

Die erfindungsgemäß beanspruchten Celluloseether sind vollständig in Wasser löslich, verbessern die superabsorbierenden Eigenschaften bei Einsatz als Pulver entscheidend, zeigen in Lösung eine gegenüber herkömmlichen Produkten geänderte Rheologie (höhere elastische Anteile) und können nach dem unten beschriebenen Verfahren entweder durch Modifizierung eines Celluloseethers oder direkt aus Cellulose hergestellt werden. Eine Beschränkung auf bestimmte Celluloseether erfolgt dabei nicht, da Art und Höhe des Veretherungsreagenzes nicht Gegenstand der vorliegenden Erfindung sind. Es können daher ionische (z. B. Carboxymethylcellulose, Sulfoethylcellulose, Carboxymethylsulfoethylcellulose u. a.) sowie nicht-ionische Celluloseether (wie z. B. Methylcellulose, Methyl-Hydroxyethylcellulose, MethylHydroxypropylcellulose, Hydroxyethylcellulose, Hydroxypropylcellulose u. a.) wie auch Mischether aus ionischen und nicht-ionischen Komponenten (z. B. Carboxymethylhydroxyethylcellulose, Carboxymethylhydroxypropylcellulose, Methylcarboxymethylcellulose, Hydroxypropyl-Sulfoethycellulose, Hydroxyethyl-Sulfoethylcellulose u. a.), sowie ionische oder nicht-ionische ternäre Mischether, die Alkyl-, Aryl- oder Hydroxyalkylgruppen sowie langkettige hydrophobe Kohlenwasserstoffreste enthalten (z. B. hydrophob modifizierte HEC oder hydrophob modifizerte HPC) durch das erfindungsgemäß beanspruchte Verfahren hergestellt werden. Auch ist es möglich, physikalische Mischungen der o. g. Celluloseether einzusetzen, wobei die erfindungsgemäß beanspruchten Celluloseether mit herkömmlichen synthetischen (Polyvinylalkoholen, Polyvinylacetaten, Polyacrylamiden u. a.) oder weiteren halbsynthetischen (Celluloseethern, Celluloseestern, Stärkeestern, Stärkeethem) oder natürlichen Polymeren (Alginaten, Stärken, Chitosan, Chitin, Holzzellstoffen, Nadelholzzellstoffen, Baumwoll-Linters, mikrokristalliner Cellulose, Lignin u. ä.) als Mischung zum Einsatz gelangen können. Der Einsatz von teilgereinigten oder salzhaltigen, sog. technischen Celluloseethem bzw. Celluloseetherabmischungen ist ebenfalls möglich. Insbesondere werden jedoch gereinigte Celluloseether, so wie sie für den Einsatz in Lebensmitteln, Pharmazeutika oder Kosmetika erforderlich sind, beansprucht.

Für die Herstellung der erfindungsgemäß beanspruchten Celluloseether ist die Konzentration an Alkali bzw. der Wassergehalt während der Alkalisierungs- und Veretherungsphase entscheidend. Die Alkalisierung kann dabei so erfolgen, dass die gesamte Menge an Alkali sowie die erforderliche Menge an Wasser zu Beginn der Alkalisierungsphase vorgelegt werden. Dabei kann das Veretherungsmittel allein oder ggf. mit zusätzlichen Mengen eines zweiten Veretherungsmittels bereits vor oder während der Alkalisierung teilweise oder vollständig vorliegen. Üblicherweise erfolgt die Zugabe der Veretherungsmittel stets nach Zugabe der gesamten Menge an Alkali oder eines Teils davon. Die wechselseitige Zugabe von Alkali und Veretherungsmittel erfolgt üblicherweise portionsweise über mehrere Schritte.

Als Alkali werden bevorzugt Natronlauge oder Kalilauge verwendet. Die Menge an Wasser kann mit dem Alkali, das z.B. in Form von Prills eingesetzt wird, zusammen oder nacheinander zum wässrig-organischen Suspensionsmittel (Slurry) eingegeben werden. Ebenso ist es möglich, einen Teil des Wassers oder die gesamte Menge an Wasser direkt mit dem Alkali als Lauge zum Slurry zu geben. Entscheidend dabei ist, dass der Gesamtwasseranteil - bezogen auf Cellulose, Suspensionsmittel, Natriumhydroxid und Veretherungsmittel (wie z.B. Cloressigsäure oder Vinylsulfonsäure o.a.)-so eingestellt wird, dass er mindestens 11 Vol.-%, insbesondere 12,5-25 Vol.-%, vorzugsweise 13 - 20 Vol.%, besonders bevorzugt 13,5 - 18 Vol.% beträgt.

Die im Vergleich zu Celluloseethern nach dem Stand der Technik veränderten Eigenschaften lassen sich nachweisen, wenn in einem Viskosimeter der Speichermodul G', der Verlustmodul G", die komplexe Viskosität $\eta^*$ und der Phasenwinkel $\delta$ bzw. der Verlustfaktor tan $\delta$ In Abhängigkeit von der Kreisfrequenz $\Omega$ bestimmt werden.

Dabei zeigt sich, dass für wässrige Lösungen der erfindungsgemäßen Celluloseether bis zu Konzentrationen von maximal 0,5 Gew.-% der Wert des Verlustfaktors tan $\delta$-weitgehen unabhängig von der Kreisfrequenz $\Omega$ unter den Werten für Celluloseether nach dem Stand der Technik liegt und bei einer Kreisfrequenz von 1 Hz kleiner als 1,0, insbesondere kleiner als 0,8 ist.

Die rheologische Charakterisierung der erfindungsgemäß beanspruchten Celluloseether, insbesondere der CMC, werden an solchen Produkten durchgeführt, deren Restsalzgehalt < 3%, insbesondere < 0,5% betrug. Als Restsalzgehalt wird der Salzgehalt im Endprodukt bezeichnet, der aufgrund von Nebenreaktionen des Veretherungsmittels mit dem Alkali oder von Alkali mit dem gegebenenfalls verwendeten Neutralisationsmittel entsteht (z.B. Natrium- oder Kaliumchlorid, Natrium- oder Kaliumacetat, Natrium- oder Kaliumglycolat u.ä.). Ferner wird zur rheologischen Charakterisierung der erfindungsgemäß beanspruchten Produkte nur entionisiertes Wasser verwendet, um etwaige Vergelungen oder Komplexierungen mit z.B. mehrwertigen Kationen zu vermeiden.

Bei den erfindungsgemäß beanspruchten und weiter unten beispielhaft beschriebenen Celluloseethern bezeichnet der durchschnittliche Substitutionsgrad (DS) die Anzahl der in der Cellulose substituierten Hydroxylgruppen pro Anhydroglucoseeinheit. Unter absolut getrockneter Substanz wird das luftgetrocknete Rohprodukt abzüglich der Feuchte verstanden.

Transmission meint den Anteil des durchstrahlenden Lichtes in Prozent des eintretenden Lichtes beim Durchgang durch eine mit einer 0,5 gew.%igen, wässrigen Celluloseetherlösung gefüllten Küvette (d = 10 mm, verwendete Wellenlänge $\lambda$ = 550 nm (Hitachi-Spektralphotometer, Modell 101, Hitachi Ltd., Tokio/Japan)).

Zur Bestimmung der vollständigen Wasserlöslichkeit wird eine Menge des lufttrockenen, gereinigten Celluloseethers eingewogen, die 500 mg absolut trockener Substanz entspricht und in 199,5 ml destilliertem Wasser gelöst wird. Diese Lösung wird vollständig durch einen 120 °C gewichtskonstant getrockneten, gewogenen Glasfiltertiegel G2 abgesaugt. Anschließend wird der Filtertiegel fünf mal mit je 100 mL destilliertem Wasser gewaschen, um Teile von anhaftendem gelösten Celluloseether zu entfernen. Der Glasfiltertiegel wird erneut bei 120 °C gewichtskonstant getrocknet und gewogen. Aus den Differenzen der Wägung wird der unlösliche Anteil bestimmt und daraus der prozentuale Anteil des löslichen Celluloseethers errechnet. Im Rahmen der Meßgenauigkeit sollen Celluloseether als vollständig löslich bezeichnet werden, die einen wasserlöslichen Anteil von mehr als 99,5 % aufweisen.

Die Erfindung wird nachfolgend anhand verschiedener Ausführungsbeispiele näher erläutert.

**Beispiele**

**Beispiel CMC 1 (Vergleichsbeispiel 1):**

Herstellung einer dem Stand der Technik entsprechenden Carboxymethylcellulose ($\equiv$ Walocel VP-C-2204 PP).

137 Teile eines feingemahlenen, gebleichten und veredelten Linterszellstoffes (Feuchte 5,3 %) werden in einem zylindrischen, in geeigneter Weise thermostatisierbaren Reaktionsgefäß, das mit einem geeigneten Rühraggregat versehen ist, eingegeben. Die Cellulose wird in 2805 mL Isopropanol suspendiert. Nach Zugabe von 295 mL Wasser, 76,8 g Natriumhydroxid-Plätzchen (Prills) wird nach Aufheizen auf 60°C 80 min lang bei dieser Temperatur alkalisiert. Anschließend werden 113,8 g Monochloressigsäure (79,8 %ig) hinzugegeben. Innerhalb von 10 min wird auf 70°C aufgeheizt und 120 min bei dieser Temperatur verethert. Das Produkt wird abfiltriert und mit einer Mischung aus 70 Teilen Methanol und 30 Teilen Wasser salzfrei gewaschen. Anschließend wird das Produkt im Umlauftrockenschrank bei 50°C getrocknet.

**Beispiel CMC 2 (Vergleichsbeispiel 2):**

In Beispiel 2 wird die Menge an Natriumhydroxid-Plätzchen auf 60,1 g reduziert, wobei alle anderen Mengen unverändert bleiben.

Für die Herstellung der erfindungsgemäß beanspruchten Carboxymethylcellulose (Muster 1) wird die in Beispiel 1 (Walocel VP-C-2204 PP) bezeichnete Rezeptur dahingehend geändert, dass der Anteil an Wasser auf 419 mL erhöht wird. Die erhaltenen Viskositäten, Trockengehalte und analytischen Kenndaten (Substitutionsgrad, Natriumchloridgehalt, Fasergehalt) sind in Tabelle 1 im Vergleich zu einem handelsüblichen Produkt vom Typ Aquasorb A 500 aufgeführt.

Tabelle 1:

| Physikalische Meßwerte der verwendeten Carboxymethylcellulosen im Vergleich | | | | | |
|---|---|---|---|---|---|
| CMC-Nr. | Viskosität [mPa.s] [1] | Trockengehalt [%] | DS[2] | NaCl [%] | Transmission [%][3] |
| CMC 1 | 6.980 | 4,8 | 0,82 | 0,52 | 99,8 |
| CMC 2 | 7.220 | 4,9 | 0,75 | 0,75 | 97,5 |
| CMC 3 (Erfindung) | 15.940 | 5,4 | 0,73 | 0,11 | 100 |
| Aquasorb A 500 [4] | 7.520 | 4,2 | 0,57 | 0,25 | 99,2 |
| [1] Brookfield, LVT, 30 UpM, Spindel 4, T = 25 °C, c = 1 % [2] Substitutionsgrad durch Carboxymethylgruppen [3] Spektralphotometer Hitachi, Modell 101, 10 mm optische Weglänge, $\lambda$ = 550 $\mu$m [4] Muster der Fa. Hercules, USA | | | | | |

Mit den oben bezeichneten Carboxymethylcellulosemustern wurden vergleichende Versuche zum Quellvermögen vorgenommen. Dabei wird so vorgegangen, dass exakt 200 mg Carboxymethylcellulose in einen Teebeutel eingegeben werden, der nachfolgend verschlossen wird. In eine Kristallisierschale werden 150 mL einer 0,9 %igen Natriumchloridlösung eingegeben (ca. 2 cm Füllhöhe). Der Teebeutel wird 10 min horizontal auf die Salzlösung gelegt. Nach Abtropfen von 1 min, wird das Quellvermögen durch Auswiegen ermittelt. Der Vorgang wird mit einem leeren Teebeutel als Nullprobe wiederholt. Die Absorption ergibt sich als:

Absorbierte Flüssigkeit in Gramm pro Gramm Muster:

$$\text{Absorption} = \frac{\text{(Auswaage) - (Nullprobe - Einwaage)}}{\text{Einwaage CMC}}$$

Hohe Werte kennzeichnen sehr gute Werte für die Wasserrückhaltung. Tabelle 2 gibt die Ergebnisse zusammenfassend wieder. Die Muster wurden vor der Austestung durch Mahlung und Siebung auf eine Sieblinie von 100 % < 2 mm, 100 % < 0,5 mm und 80 % < 0,075 mm eingestellt. Die Aufnahme des Quellvermögens erfolgte einmal am nativen, d. h. thermisch unbelasteten Material, und zum anderen nach thermischer Belastung (15 min) bei 180 °C.

Tabelle 2:

| Quellversuche im Vergleich | | |
|---|---|---|
| Muster | Absorption [1] [g/g] | Absorption nach 180°C (15 min) [g/g] |
| CMC 1 | 22,3 | 23,5 |
| CMC 2 | 24,9 | 20,1 |
| CMC 3 (Erfindung) | 42,0 | 43,3 |

(fortgesetzt)

| Quellversuche im Vergleich | | |
|---|---|---|
| Muster | Absorption [1] [g/g] | Absorption nach 180°C (15 min) [g/g] |
| Aquasorb A 500 [2] | 24,1 | 26,6 |
| [1] Doppelbestimmung Teebeutel, Typ KC 542, 76 mm Breite [2] Muster der Fa. Hercules (s. Tabelle 1) | | |

Es zeigt sich, dass das CMC-Muster 3 gegenüber üblicherweise verwendeten Handelsprodukten (z. B. CMC 1 und Aquasorb A 500) sowohl als thermisch unbelastetes Produkt als auch nach Temperierung bei 180 °C verbesserte superabsorbierende Eigenschaften aufweist.

Mit den oben bezeichneten Produkten wurden ferner Gelfestigkeiten bestimmt. Dabei wurden unterschiedlich konzentrierte Lösungen angesetzt und Lösungen gleicher Konzentration mit einem Texture Analyser hinsichtlich der Festigkeit untersucht. Tabelle 3 gibt das Ergebnis wieder.

Tabelle 3:

| Gelfestigkeiten im Vergleich [1] | | |
|---|---|---|
| Produkt | Festigkeit [g] | Konzentration [%] |
| CMC 1 | 25 | 1,25 |
|  | 45 | 1,50 |
|  | 89 | 2,00 |
| CMC 2 | 19 | 1,25 |
|  | 31 | 1,50 |
|  | 54 | 2,00 |
| CMC 3 (Erfindung) | 36 | 1,25 |
|  | 56 | 1,50 |
|  | 99 | 2,00 |
| Aquasorb A 500 | 20 | 1,25 |
|  | 35 | 1,50 |
|  | 55 | 4,00 |
| 1) Eindringtiefe 10 mm, MeßkörperTA 11; Geschwindigkeit 1,0 mm/s Gerät: LFRA-Texture-Analyser, Fa. Stevens | | |

Der Vergleich der Proben untereinander zeigt, dass die Unterschiede in der Festigkeit der Gele mit ansteigender Konzentration immer ausgeprägter werden. Die erfindungsgemäß beanspruchten Produkte weisen jedoch auch noch bei Konzentrationen von < 2 % deutlich höhere Festigkeiten auf als die Vergleichsmuster.

Um das Wasserrückhaltevermögen der gelösten Superabsorber unter anwendungstechnisch relevanten Bedingungen zu testen, wurde in eine Kristallisierschale mit einem Durchmesser von 5,5 cm und einer Höhe von 1,2 cm eine Lösung des entsprechenden CMC-Musters eingegeben (Füllhöhe bis zum Rand). Zuvor wurden aus handelsüblichem Haushaltstuch (Marke Zewa) zwei kreisrunde Stücke geschnitten, die einen etwas größeren Durchmesser als die Kristallisierschale hatten. Beide Stücke wurden exakt übereinander gelegt und zusammen auf die Produktoberfläche gelegt. Auf diese Anordnung wurde zur Stabilisierung eine Platte mit einem Gewicht von 114 g gelegt, die einen guten Kontakt der beiden Haushaltstuchstücke zu dem Produkt gewährleisten. Danach wurde die Anordnung um 180 ° gewendet. Zwischen Auflegen der Hauhaltstuchstücke und dem Wenden der Anordnung vergingen 5 s. Nach 1 min wurde durch eine Differenzwägung die aufgenommene Wassermenge des Haushaltstuchstückes bestimmt, das nicht im direkten Kontakt mit dem Produkt gestanden hatte. Die relative Wasseraufnahme in Prozent gibt Tabelle 4 wieder.

Tabelle 4:

| Wasserrückhaltevermögen von gelösten Superabsorbern unter Druck im Vergleich | |
|---|---|
| Muster | Relative Wasseraufnahme [%][1] |
| CMC 1 | 48 $\pm$ 3 |

(fortgesetzt)

| Wasserrückhaltevermögen von gelösten Superabsorbern unter Druck im Vergleich | |
|---|---|
| Muster | Relative Wasseraufnahme [%][1] |
| CMC 2 | 62 ± 3 |
| CMC 3 (Erfindung) | 10 ± 3 |
| Aquasorb A 500 | 20 ± 3 |
| [1] Wasseraufnahme für Haushaltstuch (Marke Zewa): Benetzte Papierfläche 24 cm$^2$; Mittelwerte aus 5 Messungen | |

Wie oben bereits beschrieben, gelten Superabsorber auf Polyacrylatbasis als biologisch nicht oder nur sehr schwer abbaubar. Die erfindungsgemäß beanspruchten Produkte gelten als deutlich besser abbaubar als herkömmliche Celluloseether. Zur Bestimmung der biologischen Abbaubarkeit der vorgenannten Produkte wurde das Zahn-Wellens-Verfahren verwendet. Das Produkt mit der oben aufgeführten Bezeichnung CMC 3 (Erfindung) wurde mit einem herkömmlichen Carboxymethylcelluloseether vom Typ Walocel VP-C-2204 PP (Handelsprodukt der Wolff Walsrode AG) hinsichtlich der Abbaubarkeit nach DIN EN 29888 untersucht. Die Konzentration der Testsubstanzen im Test betrug 0,5 g/L (entsprechend einem DOC-Gehalt von ca. 200 mg/L). Als Referenzsubstanz für beide Produkte wurde Diethylenglycol verwendet, das den höchsten Abbaugrad aufweist und damit als "Biologisch abbaubar" zu bewerten ist. Die Konzentration des Inokulums (Belebtschlamm der Kläranlage Bomlitz) in den Ansätzen betrug ca. 0,3 g/L. Die DOC-Gehalte wurden photometrisch mittels Küvettentest (Fa. Dr. Lange) bestimmt. Das Ergebnis in Tabelle 5 zeigt, dass die erfindungsgemäß beanspruchte CMC gegenüber herkömmlichen Celluloseethern deutlich besser abbaubar ist.

Tabelle 5:

| Ergebnisse zur DOC-Bestimmung und zur biologischen Abbaubarkeit [1] | | | | | |
|---|---|---|---|---|---|
| Muster | Anfangskonzentration | nach 7 Tagen | | nach 28 Tagen | |
| | DOC [mg/L] | DOC [mg/L] | Abbau [%] | DOC [mg/L] | Abbau [%] |
| Blindwert | 1,8 | 1,7 | - | 2,6 | - |
| Referenz [2] | 218 | 184 | 16 | 49,9 | 78 |
| CMC 3 (Erfindung) | 192 | 148 | 23 | 129 | 34 |
| Walocel VP-C-2204 PP | 154 | 154 | 0 | 153 | 1 |
| [1] DOC = gelöster organisch gebundener Kohlenstoff [2] Diethylenglykol | | | | | |

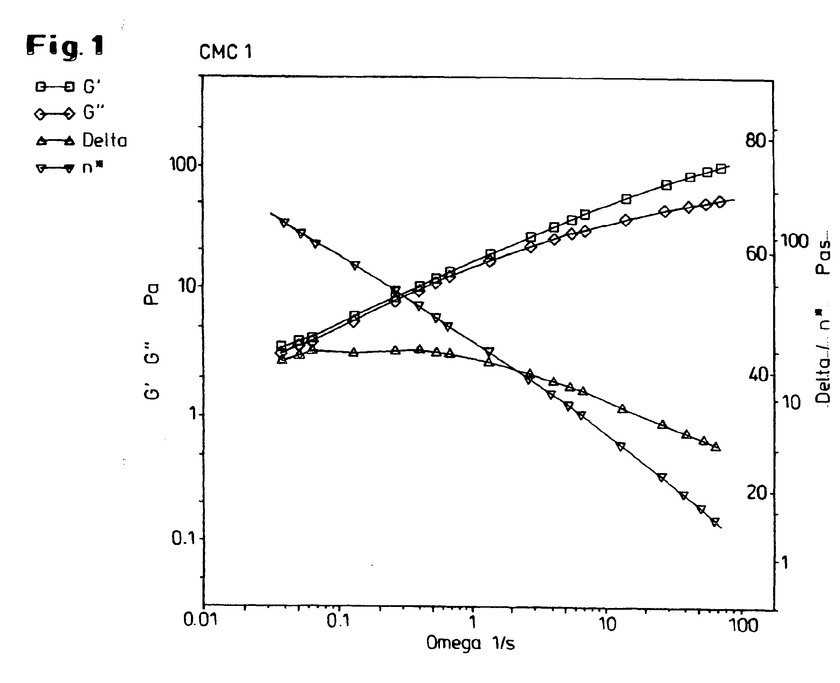

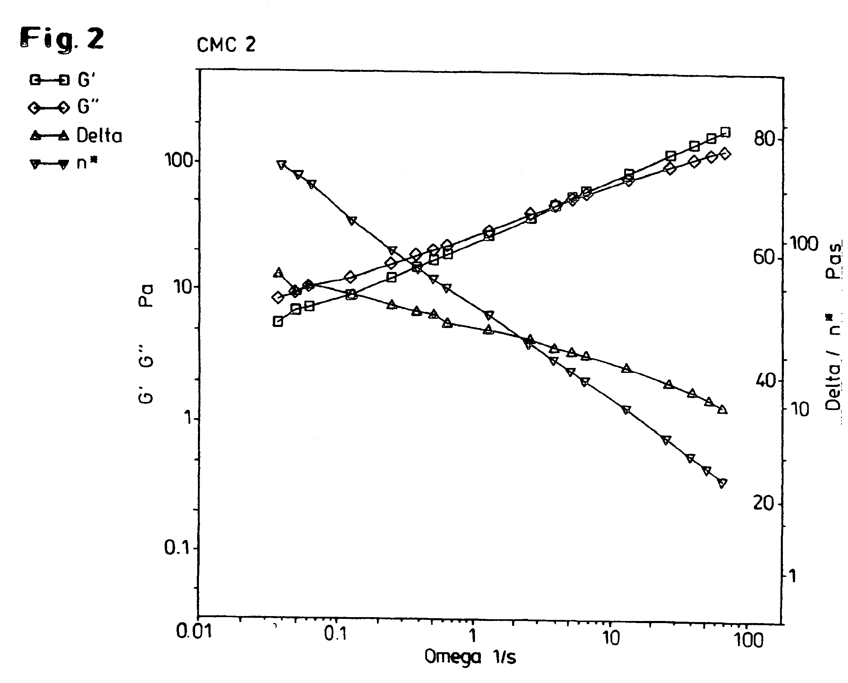

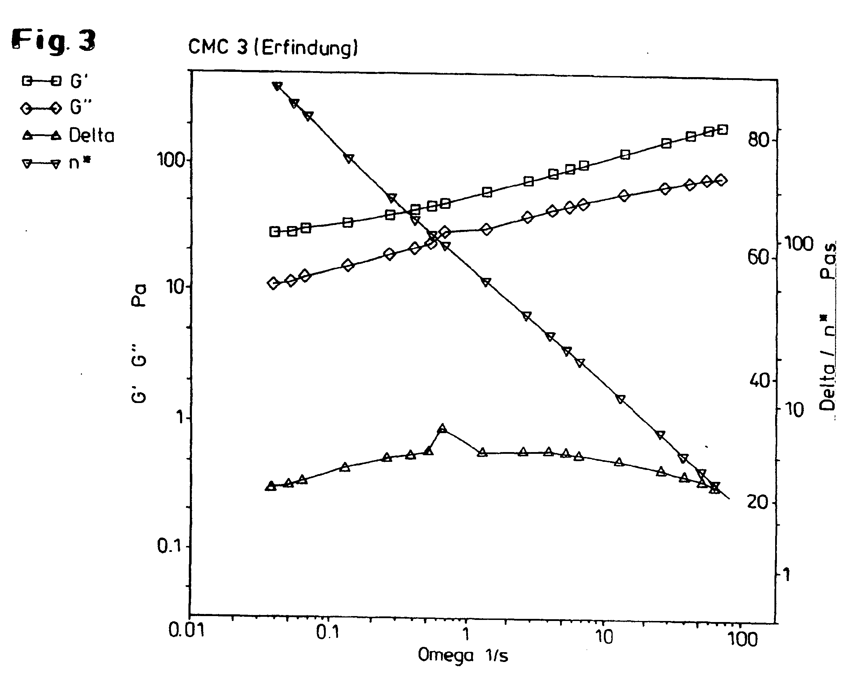

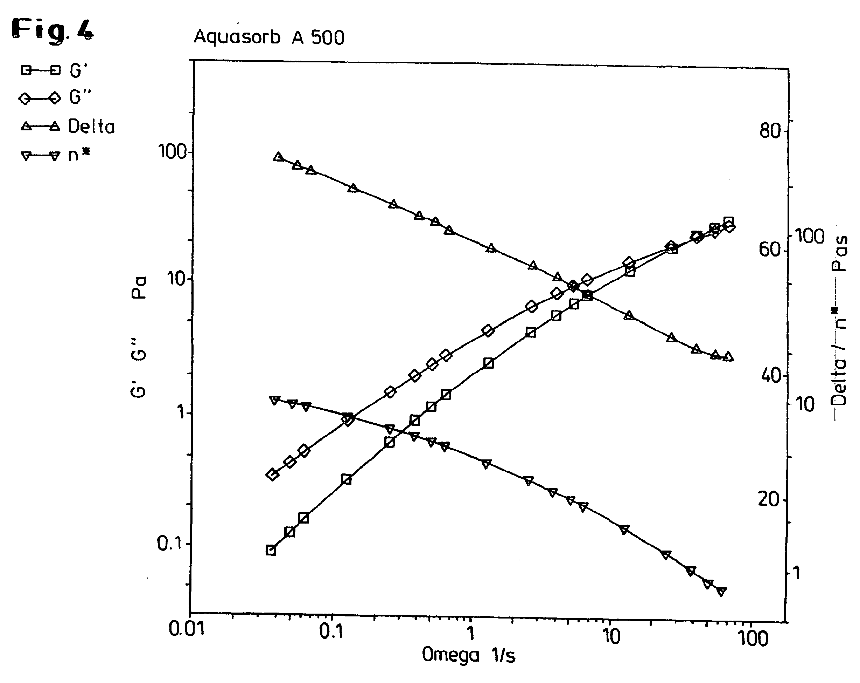

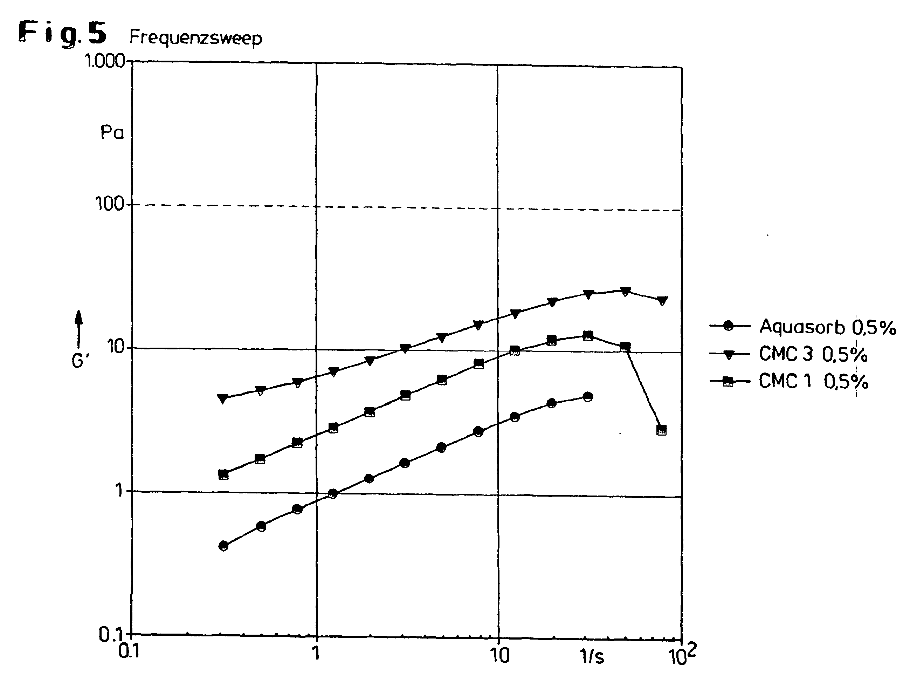

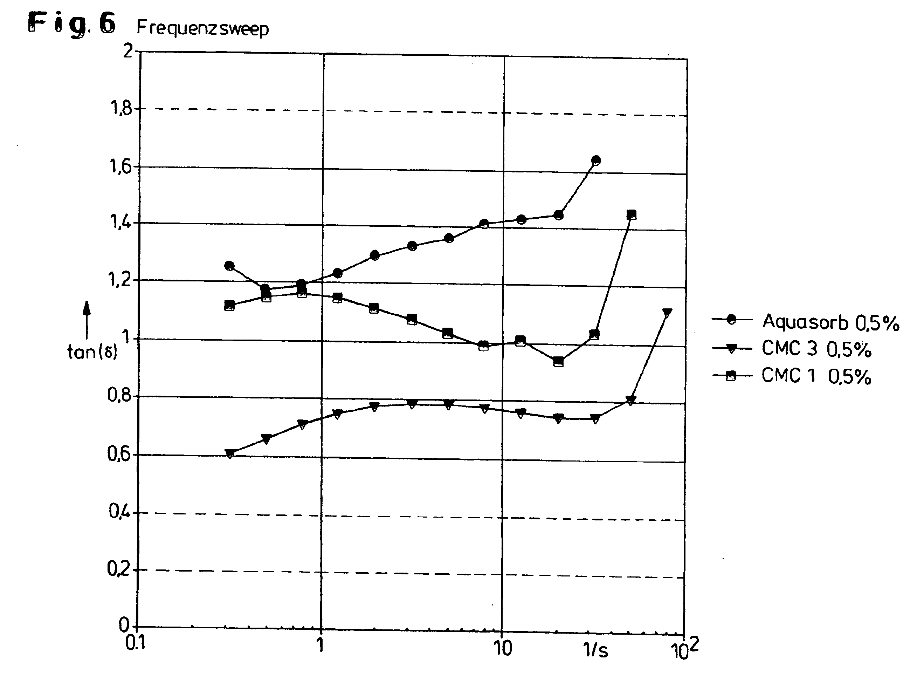

Mit einem schubspannungsgesteuerten Viskosimeter (CS 50 der Fa. Bohlin bzw. einem Rotationsviskosimeter der Fa. Physica, Stuttgart, (Typ UDS 200; Abb. 5 und 6)) wurden die viskoelastischen Eigenschaften der Produkte bei einer Konzentration von c= 1 Gew.-% und einer Temperatur von 25 °C in Abhängigkeit von der Kreisfrequenz $\Omega$ gemessen. In den Abbildungen 1 - 4 wurde der Speichermodul G', der Verlustmodul G", die komplexe Viskosität $\eta^*$ und der Phasenwinkel $\delta$ in Abhängigkeit von der Kreisfrequenz $\Omega$ der einzelnen Produkte dargestellt.

Die Ergebnisse, die in den Abbildungen 1 - 4 aufgeführt sind, zeigen, dass sich die Produkte hinsichtlich der viskoelastischen Eigenschaften sehr deutlich voneinander unterscheiden. Das Fließverhalten des Aquasorb-Musters ist bei niedrigen Frequenzen durch viskoses Fließen gekennzeichnet (G" > G'; der viskose Anteil dominiert über den elastischen Anteil). Mit zunehmender Frequenz steigt $G^1$ stärker an als G", so dass sich die beiden Kurven in einem Punkt schneiden. Oberhalb dieses Schnittpunktes wird das Verhalten der Probe durch den elastischen Anteil bestimmt. Der beschriebene Verlauf ist typisch für eine konzentrierte Polymerlösung. In Bezug auf das viskoelastische Verhalten (Moduldaten, Verlustfaktor) zeigen die Muster CMC 1 und CMC 2 ähnliche Eigenschaften. Die Gel-Festigkeiten der Lösungsstruktur bzw. des Verhakungsnetzwerkes liegen jedoch deutlich unterhalb der des Aquasorb-Musters (G'-Werte deutlich kleiner).

Die erfindungsgemäß beanspruchte CMC Nr. 3 unterscheidet sich rheologisch vollkommen von den anderen Proben. Fast über den gesamten Frequenzbereich liegt der elastische Anteil G' über dem viskosen Anteil G". Die Probe zeigt rein elastisches Verhalten. Das Produkt ist durch einen sehr flachen Anstieg des Speichermoduls und dem im gesamten Frequenzbereich deutlich höheren Speichermodul G' gekennzeichnet (s. auch Abbildung 5).

In Abbildung 6 ist der Verlustfaktor (tan $\delta$) gegen die Kreisfrequenz $\Omega$ als Frequenzsweep für 0,5 Gew.-%ige wässrige Lösungen der in Tabelle 1 bezeichneten CMC-Muster aufgeführt. Aufgrund der hohen elastischen Anteile der erfindungsgemäß beanspruchten Celluloseether, insbesondere von CMC (s. Abbildung 6, CMC Nr. 3), liegen die Werte für den Verlustfaktor praktisch unabhängig von der Kreisfrequenz deutlich unterhalb derjenigen der VergleichsMuster.

**Patentansprüche**

1. Weitgehend faserfreie Celluloseether, **dadurch gekennzeichnet, dass** der Gesamtwasseranteil bei der Herstellung der Celluloseether bei 11 bis 28 Vol.-%, bezogen auf Cellulose, Suspensionsmittel, Natriumhydroxid und Veretherungsmittel, liegt und zur Herstellung der Celluloseether Mengen von Alkali von mindestens 1,8 Mol bis 2,6 Mol / Mol Glukoseeinheit verwendet werden und wässrige Lösungen in Konzentrationen von maximal 0,5 Gew.-% Werte für den Verlustfaktor (tan $\delta$) von < 1,0 bei einer Kreisfrequenz von 1 Hz zeigen.

2. Celluloseether nach Anspruch 1, **dadurch gekennzeichnet, dass** es sich bei den Celluloseethern um Carboxymethylcelluloseether, Sulfoethylcelluloseether, Carboxymethylsulfoethylcelluloseether, Methylcelluloseether, Methylhydroxyethylcelluloseether, Methylhydroxypropylcelluloseether, Hydroxyethylcelluloseether oder Hydroxypropylcelluloseether handelt.

3. Celluloseether nach mindestens einem der vorgenannten Ansprüche, **dadurch gekennzeichnet, dass** es sich bei den Celluloseethern um Carboxymethylcelluloseether oder Sulfoethylcelluloseether handelt.

4. Celluloseether nach mindestens einem der vorgenannten Ansprüche, **dadurch gekennzeichnet, dass** zur Herstellung der Celluloseether wässrig-organische Suspensionsmittel, wie z. B. Isopropanol-Wasser-, Aceton-Wasser-, Methanol-Wasser-, Ethanol-Wasser- oder tertiär-Butanol-Wasser-Gemische oder binäre oder ternäre Gemische der vorgenannten Suspensionsmittel mit Wasser verwendet werden.

5. Celluloseether nach mindestens einem der vorgenannten Ansprüche, insbesondere Carboxymethylcelluloseether, **dadurch gekennzeichnet, dass** für die Herstellung der Carboxymethylcellulose eine Menge an Monochloressigsäure von 0,4 bis 2,5 Mol, vorzugsweise von 0,5 bis 1,8 Mol, insbesondere von 0,6 bis 1,5 Mol pro Mol Glukose eingesetzt wird.

6. Celluloseether, insbesondere Carboxymethylcelluloseether (CMC) mit superabsorbierenden Eigenschaften nach mindestens einem der vorgenannten Ansprüche, **dadurch gekennzeichnet, dass** die CMC **gekennzeichnet ist durch** folgende Eigenschaften:

   a) Saugfähigkeit mindestens 30 g Flüssigkeit/Gramm Celluloseether
   b) eine **durch** Mahlung und Siebung eingestellte Sieblinie von 100 % < 2 mm, 100 % < 0,5 mm und mindestens 80 % < 0,075 mm
   c) als Lösung eine Viskosität von mindestens 10.000 mPa·s, insbesondere > 12.000 mPa·s, besonders bevorzugt von > 13.000 mPa·s (Brookfield, LVT, 30 UpM, Spindel 4, T = 25°C, c = 1 Gew.-%)
   d) einen wasserlösliche Anteil von > 99,0 % (0,25 %ige Lösung) eine Transmission von > 99,9 % (0,5 Gew.-%ige wässrige Lösung; Spektralphotometer Hitachi, optische Weglänge d = 10 mm, $\lambda$ = 550 nm)

7. Verwendung von Celluloseethern nach mindestens einem der vorgenannten Ansprüche als Hilfsmittel für Anwendungen im Kosmetik-, Pharma- und Lebensmittelbereich sowie zur Herstellung von Anstrichmitteln (Dispersions- und Silikatfarben) sowie im Tiefbau.

**Claims**

1. Substantially fibre-free cellulose ethers, **characterised in that** the total water content during the production of the cellulose ethers is 11 to 28 vol.%, relative to cellulose, suspension medium, sodium hydroxide and etherification agent, and alkali quantities of at least 1.8 mol to 2.6 mol glucose unit are used for the production of the cellulose ethers and aqueous solutions with maximum concentrations of 0.5 wt.% exhibit loss factor (tan $\delta$) values of < 1.0 at an angular frequency of 1 Hz.

2. Cellulose ethers according to claim 1, **characterised in that** the cellulose ethers are carboxymethyl cellulose ethers, sulfoethyl cellulose ethers, carboxymethyl sulfoethyl cellulose ethers, methyl cellulose ethers, methyl hydroxyethyl cellulose ethers, methyl hydroxypropyl cellulose ethers, hydroxyethyl cellulose ethers or hydroxypropyl cellulose ethers.

3. Cellulose ethers according to at least one of the aforementioned claims, **characterised in that** the cellulose ethers are carboxymethyl cellulose ethers or sulfoethyl cellulose ethers.

4. Cellulose ethers according to at least one of the aforementioned claims, **characterised in that** aqueous-organic suspension media, such as e.g. mixtures of isopropanol and water, acetone and water, methanol and water, ethanol and water or tert.-butanol and water or binary or ternary mixtures of the aforementioned suspension media with water are used for the production of the cellulose ethers.

5. Cellulose ethers according to at least one of the aforementioned claims, in particular carboxymethyl cellulose ethers, **characterised in that** an amount of monochloroacetic acid of 0.4 to 2.5 mol, preferably 0.5 to 1.8 mol, in particular 0.6 to 1.5 mol/mol glucose, is used for the production of the carboxymethyl cellulose.

6. Cellulose ethers, in particular carboxymethyl cellulose ethers (CMCs) having superabsorbent properties according to at least one of the aforementioned claims, **characterised in that** the CMC is **characterised by** the following properties:

   a) an absorbency of at least 30 g liquid / gram cellulose ether
   b) b) a grading curve, as adjusted by grinding and screening, of 100 % < 2 mm, 100 % < 0.5 mm and at least 80 % < 0.075 mm
   c) a viscosity as solution of at least 10,000 mPa·s, in particular > 12,000 mPa·s, particularly preferably > 13,000 mPa·s (Brookfield, LVT, 30 rpm, spindle 4, T - 25°C, c = 1 wt.%)
   d) a water-soluble fraction of > 99.0 % (0.25 % solution), a transmission of > 99.9 % (0.5 wt.% aqueous solution; Hitachi spectrophotometer, optical path length d = 10 mm, $\lambda$ = 550 nm).

7. Use of cellulose ethers according to at least one of the aforementioned claims as auxiliary substances for applications in the cosmetics, pharmaceutical and food sectors and for the production of paints (emulsion and silicate paints) and in civil and underground engineering.

**Revendications**

1. Ethers cellulosiques pratiquement exempts de fibres et **caractérisés en ce que**, à leur préparation, la proportion totale d'eau est de 11 à 28 % en volume par rapport à la cellulose, au milieu de suspension, à l'hydroxyde de sodium et à l'agent éthérifiant et les quantités d'alcali utilisées sont d'au moins 1,8 à 2,6 mol par mole de motif de glucose, et les solutions aqueuses de ces éthers cellulosiques à des concentrations de 0,5 % en poids au maximum présentent des valeurs du facteur de perte (tg $\delta$) < 1,0 à une fréquence de rotation de 1 Hz.

2. Ethers cellulosiques selon de la revendication 1, **caractérisés en ce qu'**ils consistent en éthers de carboxyméthylcellulose, éthers de sulfoéthylcellulose, éthers de carboxyméthylsulfoéthylcellulose, éthers de méthylcellulose, éthers de méthylhydroxyéthylcellulose, éthers de méthylhydroxypropylcellulose, éthers d'hydroxyéthylcellulose ou éthers d'hydroxypropylcellulose.

3. Ethers cellulosiques selon au moins une des revendications qui précèdent, **caractérisés en ce qu'**il s'agit d'éthers de carboxyméthylcellulose ou d'éthers de sulfoéthylcellulose.

4. Ethers cellulosiques selon au moins une des revendications qui précèdent, **caractérisés en ce que**, pour leur préparation, on a utilisé des milieux de suspension hydro-organiques, par exemple des mélanges isopropanol-eau, acétone-eau, méthanol-eau, éthanol-eau ou tert-butanol-eau ou des mélanges binaires ou ternaires des mêmes milieux de suspension et d'eau.

5. Ethers cellulosiques selon au moins une des revendications qui précèdent, en particulier éthers de carboxyméthylcellulose, **caractérisés en ce que**, pour leur préparation, on a utilisé l'acide monochloracétique en quantité de 0,4 à 2,5 mol, de préférence de 0,5 à 1,8 mol et plus spécialement de 0,6 à 1,5 mol par mole de glucose.

6. Ethers cellulosiques, plus spécialement éthers de carboxyméthylcellulose (CMC) à propriétés superabsorbantes selon au moins une des revendications qui précèdent, **caractérisés en ce que** les CMC se caractérisent par les propriétés suivantes :

a) capacité d'absorption : au moins 30 g de liquide/gramme de l'éther cellulosique

b) ligne de tamisage, réglée par broyage et tamisage de 100 % < 2 mm, 100 % < 0,5 mm et au moins 80 % < 0,075 mm

c) une viscosité en solution d'au moins 10 000 mPa·s, de préférence > 12 000 mPa·s et dans les meilleures conditions > 13 000 mPa·s (viscosimètre Brookfield, LVT, 30 tr/min, broche n° 4, T = 25°C, c = 1 % en poids)

d) une fraction soluble dans l'eau supérieure à 99,0 % (en solution à 0,25 %) un coefficient de transmission supérieur à 99,9 % (solution aqueuse à 0,5 % en poids; spectrophotomètre Hitachi, trajet optique d = 10 mm, $\lambda$ = 550 mm).

7. Utilisation des éthers cellulosiques selon au moins une des revendications qui précèdent en tant que produits auxiliaires dans des applications en cosmétique, en pharmacie et dans les produits alimentaires ou pour la préparation de produits de revêtement (peintures en dispersion ou peintures au silicate) ainsi que dans les travaux publics.

Fig. 1

CMC 1

G'
G''
Delta
n*

Omega 1/s

Delta / n* — Pas

G', G'' Pa

Fig. 2

CMC 2

**Fig. 3**

CMC 3 (Erfindung)

G'
G"
Delta
n*

EP 1 025 130 B2

Fig. 4

Aquasorb A 500

**Fig.5** Frequenzsweep

Legend:
- Aquasorb 0,5%
- CMC 3 0,5%
- CMC 1 0,5%

Fig. 6  Frequenzsweep

Aquasorb 0,5%
CMC 3 0,5%
CMC 1 0,5%

tan(δ)

2
1,8
1,6
1,4
1,2
1
0,8
0,6
0,4
0,2
0

0,1   1   10   1/s   10²

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2715124 A
- JP 04161431 A
- JP 63037143 A
- US 4952550 A
- WO RD349022 A
- EP 0131090 A
- US 4110226 A
- US 3574188 A
- EP 0056360 A
- DE 3929400
- DE 4328190 A1
- DE 4206857 A1
- EP 0201895 B1
- EP 0080678 A
- EP 0161607 A
- EP 0126959 A
- US 4547570 A
- SU 553253 B
- CA 871977